# EUROPEAN PATENT APPLICATION

(11) **EP 3 685 778 A1**
(43) Date of publication of application: **29.07.2020**
(21) Application number: 19153655.6
(22) Date of filing: 25.01.2019
(51) Int. Cl.: A61B 18/02, A61B 18/12, A61B 18/14, A61B 34/10, A61B 34/32, A61B 90/00, A61B 18/04, A61B 17/34, B25J 9/16, A61B 34/20, A61B 18/00

(54) **APPARATUS FOR DETERMINING A POSITION OF A TEMPERATURE PROBE DURING A PLANNING FOR AN ABLATION PROCEDURE**

(71) Applicant: Koninklijke Philips N.V., 5656 AG Eindhoven (NL)
(72) Inventor: HAUTVAST, Guillaume Leopold Theodorus Frederik, 5656 AE Eindhoven (NL)
(74) Representative: de Haan, Poul Erik

(57) **Abstract**

The invention refers to an apparatus allowing to determine a planned position of a temperature probe during a planning for an ablation procedure. The apparatus comprises an ablation plan providing unit 121 for providing an ablation plan, wherein the ablation plan comprises geometric information of an ablation region and of a protection region. The protection region should be protected from ablation. The geometric information comprises information on a position and shape of a respective region. A temperature probe position determination unit 122 is adapted to determine a planned position of the temperature probe, wherein the planned position of the temperature probe is determined based on the geometric information of the ablation region and of the protection region. The apparatus allows to increase the protection of regions that should be protected, like a specific organ, such that the security of an ablation procedure for a patient can also be increased.

## Description

### FIELD OF THE INVENTION

The invention relates to an apparatus, a method and a computer program for determining a planned position of a temperature probe during a planning for an ablation procedure, and to an ablation procedure planning and guidance system for planning and guiding an ablation procedure comprising the apparatus.

### BACKGROUND OF THE INVENTION

During an ablation procedure, ablation probes are inserted into a target region, for instance, a tumor region, in a patient, wherein the ablation probes provide heat or cold to the target region to ablate the target region. In many cases, the target region is surrounded by tissue that should not be ablated, for instance, organ tissue, due to its important biological function. To protect the tissue regions that should not be ablated, for instance, an organ in the vicinity of the target region, usually temperature probes are inserted that measure the temperature during the ablation procedure and thus can give a physician an idea on the temperature distribution and on the temperature development in the ablation region and the region that should be protected. At present, these temperature probes are inserted by the physician based on general rules and on his/her experience. Thus, the success of protecting a region that should not be ablated during an ablation procedure strongly depends on the skills of a physician performing the ablation procedure.

### SUMMARY OF THE INVENTION

It is an object of the present invention to provide an apparatus, a method and a computer program allowing to determine a planned position of a temperature probe during a planning for an ablation procedure such that a protection of an organ at risk during an ablation procedure can be improved.

In a first aspect of the present invention an apparatus for determining a planned position of a temperature probe during a planning for an ablation procedure is presented, wherein the apparatus comprises a) an ablation plan providing unit for providing an ablation plan, wherein the ablation plan comprises geometric information of an ablation region and of a protection region, wherein the protection region should be protected from ablation during the ablation procedure, wherein the geometric information comprises information on a position and shape of a respective region, and b) a temperature probe position determination unit for determining a planned position of the temperature probe that should be positioned to protect the protection region during the ablation procedure, wherein the position of the temperature probe is determined based on the geometric information of the ablation region and of the protection region.

Since the temperature probe position determination unit determines a planned position of a temperature probe that should be positioned to protect the protection region based on geometric information of the ablation region and of the protection region comprising a position and shape of the respective region, the planned position of the temperature probe can be determined such that, if the temperature probe is positioned at the planned position, it can measure the temperature in a region that allows a user like a physician to estimate very accurately if the protection region, i.e. a region in the vicinity of the target region that should be protected, is in danger of being ablated. Accordingly, the apparatus allows to increase the protection of regions that should be protected, like a specific organ, such that the security of an ablation procedure for a patient can also be increased.

The ablation plan providing unit, that is adapted for providing an ablation plan, can be, for instance, a storage unit for storing the ablation plan. Alternatively, the ablation plan providing unit can be connected to a storage unit storing the ablation plan. Moreover, the ablation plan providing unit might also be directly connected to an ablation plan determination unit that determines an ablation plan, wherein the ablation plan determination unit provides the ablation plan to the ablation plan providing unit. The ablation plan comprises at least geometric information of an ablation region and of a protection region, wherein the geometric information comprises information on a position and shape of the respective region, i.e. information on a position and shape of the ablation region and information on a position and shape of the protection region. The geometric information can be acquired, for instance, based on a medical image of the region of interest that should be undergoing an ablation procedure, wherein the medical image can be an image like an ultrasound image, a CT image, a PET image, etc. Alternatively, the geometric information might be acquired based on pre-knowledge on the region of interest, for instance, based on generally known positions and shapes of respective structures in the region of interest. Moreover, the position and shape can be provided by a user, for instance, by delineating and segmenting an ablation region and a protection region in a provided medical image, or automatically, for instance, by an automatic segmentation of a medical image of the region of interest. The ablation region refers to a region that should be ablated, for instance, a tumor with an additional safety margin that ensures that the whole tumor is ablated. The protection region refers to a region that should be protected, for instance, because it comprises an organ with an important function for the patient. In the following, such an organ will be called an organ at risk. The protection region can, additionally or alternatively to an organ at risk, comprise tissue, like muscle tissue or bone tissue, that should not be ablated. Further, the protection region can comprise also a safety margin to ensure that an organ at risk is safe from the ablation procedure. The ablation plan can further comprise additional information, for instance, an already planned position of an ablation probe or information on the general procedure planned for the ablation procedure.

The temperature probe position determination unit is adapted to determine a planned position of the temperature probe that should be positioned to protect a protection region during the ablation procedure. The temperature probe is configured to measure a temperature of tissue surrounding the temperature probe when it is positioned inside the patient during an ablation procedure. When the temperature probe is positioned during an ablation procedure to protect the protection region, the temperature probe measures the temperature of the tissue in contact with the temperature probe and provides the measured temperatures to a user like a physician, wherein the provided temperature allows the user to estimate if the protection region is in danger of being ablated. Alternatively, the temperature measured by the temperature probe might be provided to an alarm system that sounds an alarm during the ablation procedure when the temperature measured by the temperature probe exceeds a predetermined threshold that indicates that the protection region might be ablated.

The planned position of the temperature probe is determined based on the geometric information of the ablation region and the geometric information of the protection region included in the ablation plan. Accordingly, the temperature probe position determination unit can determine the planned position of the temperature probe such that, in accordance with the geometric information, the temperature probe is positioned in the optimal position to provide its protective function. This planned position can be, for instance, between the ablation region and the protection region such that a user knows when a temperature in the region between the ablation region and the protection region increases. The planned position of the temperature probe might also be determined such that the temperature probe is positioned near the ablation region to monitor the temperature development in the surroundings of the ablation region such that the ablation procedure can be stopped when the temperature in the surroundings of the ablation region becomes too high. Alternatively, the temperature probe might be positioned near the protection region such that temperature changes near the protection region can be closely monitored and the ablation procedure might be stopped if the temperature development in the protection region is not in accordance with a plan for protecting the protection region. Preferably, the temperature probe position determination unit is adapted to determine an intersection point between the shortest path and the protection region as planned position of the temperature probe.

In an embodiment, the temperature probe position determination unit is adapted to determine a shortest path between the ablation region and the protection region and to determine the planned position of the temperature probe along the shortest path. Since the shortest path between the ablation region and the protection region is determined by the temperature probe position determination unit and the planned position of the temperature probe is determined along this shortest path, it can be ensured that the temperature probe monitors the temperature in a region comprising the highest risk to be involuntarily ablated when the temperature probe is positioned at the planned position during the ablation procedure. Thus, the protection of the protection region can be further improved. The shortest path between the ablation region and the protection region can be determined, for instance, based on the shapes and positions of the ablation region and the protection region by using known geometric methods. Moreover, computer simulations might be used to determine the shortest path between the ablation region and the protection region. The shortest path can refer to the shortest path between a surface or boundary of the ablation region defined by the shape of the ablation region and a surface or boundary of the protected region defined by the shape of the protected region. Alternatively, the shortest path can refer to a shortest path between a center point of the ablation region and a center point of the protected region.

In an embodiment, the ablation plan further comprises a position of a grid template comprising a plurality of grid points through which a temperature probe can be inserted, wherein the temperature probe position determination unit is adapted to further determine the planned position of the temperature probe based on the position of the grid template. In many ablation procedures grid templates are used to increase an accuracy of positioning probes like target probes or temperature probes inside a patient while at the same time increasing the simplicity of the procedure. Generally, grid templates refer to a grid comprising a plurality of grid points through which the probes used during the procedure can be inserted into the patients. The grid points can be provided on the grid template in any kind of configuration. For instance, the grid points can be uniformly and evenly spaced in a rectangular or circular pattern. In other examples the grid points might be unevenly or even arbitrarily distributed on the grid template. Generally, the position of the grid points on the grid template is known and thus the position of the grid points is defined by the position of the grid template. The position of the grid template provided by the ablation plan refers to a position of the grid template with respect to the patient, i.e. with respect to the ablation region and the protection region. The grid points of the grid template can be configured such that a temperature probe that is positioned through a grid point follows a predetermined path determined by the configuration of the grid point. For instance, the grid point can be configured such that a temperature probe inserted through the grid point follows a path perpendicular to the grid template. But, also other paths, for instance, tilted paths with respect to the grid template can be provided by the grid points. The temperature probe position determination unit is then adapted to determine the planned position of the temperature probe based on the position of the grid template. Accordingly, the temperature probe position determination unit can determine the planned position of the temperature probe such that this planned position of the temperature probe can be reached by a temperature probe inserted through the grid points of the grid template.

Preferably, the position determination unit is adapted to determine the planned position of the temperature probe based on the position and configuration of the grid points of the grid template. This ensures that the user can position the temperature probe during the ablation procedure very accurately through the grid points of the grid template. Preferably, the temperature probe position determination unit is adapted to determine a planned position of the temperature probe comprising a grid point of the grid template through which the temperature probe should be positioned. Since the planned position of the temperature probe is mainly determined by a position and configuration of a grid point through which the temperature probe is inserted, the ablation procedure can be simplified for a user if the planned position provided comprises information on a specific grid point through which the temperature probe should be inserted.

In an embodiment, the temperature probe position determination unit is adapted to determine an intersection point between the shortest path and the protection region, and to determine as planned position of the temperature probe a position of a nearest grid point with respect to the intersection point. The intersection point between the shortest path and the protection region can be defined, for instance, as a point at which the shortest path crosses a surface or boundary of the protection region. If the position of the temperature probe is then determined as being the position of a nearest grid point with respect to the intersection point, it is ensured that the temperature probe is provided as near as possible to a surface or boundary of the protection region. Since the part of the protection region that has the highest risk of being involuntarily ablated is the part of the protection region nearest to the ablation region, providing the nearest grid point to the intersection point as planned position of the temperature probe allows to closely monitor this part of the protection region when the temperature probe is positioned at the planned position. Thus, the protection of the protection region can be further improved.

In an embodiment, the ablation plan further comprises an estimated temperature development during the course of the ablation procedure for an affected region comprising the target region and the protection region, wherein the temperature probe position determination unit is adapted to determine the planned position of the temperature probe further based on the estimated temperature development. The estimated temperature development during the course of the ablation procedure included in the ablation plan can be provided, for instance, based on knowledge about an ablation probe that is planned to be used and on an energy that is planned to be used for the ablation. Moreover, more sophisticated models for numerically simulating a temperature development during the course of an ablation procedure can be used for estimating the temperature development.

The temperature development is estimated for the whole region of interest, i.e. for the whole affected region, which comprises the target region and the protection region. If such information on the estimated temperature development is available in the ablation plan, the temperature probe position determination unit is adapted to determine the planned position of the temperature probe further based on the estimated temperature development. This ensures that factors that affect the temperature in or near a protection region and that might not be reflected by the geometric information of the target region and of the protection region can be taken into account. For instance, a distribution of blood vessels in the vicinity of a certain part of the protection region might lead to an additional cooling of this part of the protection region, while in other parts of the protection region no cooling is to be expected. Thus, in such a case, the temperature probe position determination unit might determine a planned position for the temperature probe near a region of the protection region that is not affected by the additional cooling, since in this region higher temperatures are to be expected.

Preferably, the temperature probe position determination unit is adapted to determine the planned temperature probe position based on the expected temperature development such that expected temperature changes during the ablation procedure at the position of the temperature probe are higher than in the protected region. To effectively protect the protection region, i.e. to effectively provide a user with temperature information that allows him/her to react to the temperature information before the protection region is damaged by ablation, it is advantageous when the temperature changes, i.e. the increase in case of using heat for the ablation or decrease in case of using cold for the ablation, of the temperature, at the planned position of the temperature probe are always higher than the expected temperature changes in the protection region such that a security margin is provided and the user has enough time to react.

In an embodiment, the temperature probe position determination unit is adapted to determine the planned position of the temperature probe as a position for which in case of an ablation using heat the highest temperature and in case of using cold the lowest temperature is expected within a safety margin around the protected region. The safety margin around the protected region can refer to a region enveloping the protection region. The boundary of the safety margin can be determined by a constant distance to the boundary of the protection region, or the distance can be varying. The safety margin can be provided by a user together with or as part of the ablation plan, or the safety margin can be predefined, preferably preset, and provided to the temperature probe position determination unit. The position for which the highest or lowest temperature within the safety margin can be expected can be determined based on the estimated temperature development. For instance, the highest or lowest temperature can be determined by comparing all expected temperatures for all positions within the safety margin and for all times during the ablation procedure.

In an embodiment, the temperature probe position determination unit is further adapted to provide a threshold temperature based on a determined planned position of the temperature probe, wherein the threshold temperature is determined such that, in a case in which the tissue is ablated using heat, if temperatures below the threshold temperature are measured by the temperature probe positioned at the determined position, tissue in the protection region will not be damaged, or such that, in a case in which the tissue is ablated using cold, if temperatures above the threshold temperature are measured by the temperature probe positioned at the determined position, tissue in the protection region will not be damaged. The threshold temperature can be determined based on the planned position of the temperature probe, for instance, by determining a distance between the planned position of the temperature probe and the ablation region and to determine the temperature based on this distance. For instance, the temperature threshold can be decreased or increased depending on the kind of ablation with increasing distance from the ablation region. Moreover, the temperature threshold can further be determined based on knowledge of a sensitivity of the protection region with respect to damages from an increased or decreased temperature and the threshold can then be determined such that no damage is to be expected in the protection region if the temperature measured by the temperature probe is below or above the threshold.

In a preferred embodiment, the ablation plan further comprises an estimated temperature development during the course of the ablation procedure for an affected region comprising the target region and the protected region, wherein the temperature probe position determination unit is adapted to determine the temperature threshold further based on the estimated temperature development. In this embodiment the temperature threshold can be determined based on the knowledge of the temperature development at the planned position of the temperature probe and thus can be determined such that if the temperature measured at the position of the temperature probe during the ablation procedure corresponding to the planned position exceeds the expected temperature for this position, a warning can be issued to the physician.

In an embodiment, the ablation plan further comprises information on a sensitivity of the protection region, wherein the temperature probe position determination unit is further adapted to determine the planned position of the temperature probe based on the sensitivity information. The sensitivity information of the protection region can refer to a sensitivity with respect to a temperature. For instance, some organ tissues can be more sensitive than other organ tissues, for instance, some organ tissue might be damaged at lower temperatures than other organ tissues. Moreover, the sensitivity information might comprise information on how sensitive the protection region is with respect to puncturing. For instance, the information might comprise whether the protection region can be punctured by the temperature probe or not. Great blood vessels, for example, should generally not be punctured by the temperature probe, due to the great risk of complications, whereas other organs often will not be permanently damaged by the insertion of a temperature probe. The temperature probe position determination unit can then take this information into account when determining the planned position of the temperature probe. For instance, the temperature probe position determination unit can be adapted to avoid positions within the protection region if the sensitivity information includes a high sensitivity of the protection region with respect to puncturing. Moreover, the temperature probe position determination unit can be adapted to determine a planned position of the temperature further away from the protection region if the sensitivity information indicates that the protection region is particularly sensitive to heat.

In a further aspect of the invention, an ablation procedure planning and guidance system for planning and guiding an ablation procedure is presented, wherein the system comprises a) an ablation plan determination unit for determining an ablation plan comprising geometric information on an ablation region and a protection region, b) an apparatus according to claim 1 for determining a position of a temperature probe for the ablation procedure, and c) a final ablation plan providing unit for providing a final ablation plan comprising the ablation plan and the position of the temperature probe to an ablation procedure control unit for controlling the ablation procedure.

The ablation plan determination unit can be, for instance, adapted to determine the ablation plan automatically or in an interaction with the user. To determine an ablation plan, the ablation plan determination unit can comprise, for instance, an image processing unit for processing images of a region of interest, i.e. a region comprising a structure that should be ablated, and can be adapted to identify an ablation region and a protection region in the region of interest. Moreover, the ablation plan determination unit can further comprise means for simulating or calculating a temperature development in an affected region comprising the ablation region and the protection region based on, for instance, a position of an ablation probe, an energy provided by the ablation probe, tissue characteristics of the tissue in the affected area, an anatomical structure of the affected region, etc.

The final ablation plan comprises the ablation plan and the position of the temperature probe, such that, when the final ablation plan is provided to the ablation procedure control unit, the ablation procedure control unit can provide a user with all information necessary for carrying out the ablation procedure. Moreover, the final ablation plan can also comprise the temperature threshold, and the ablation procedure control unit can comprise an alarm unit for sounding an alarm if a temperature measured by the temperature probe positioned in accordance with the final ablation plan measures a temperature exceeding the previously determined temperature threshold.

In an embodiment, the system further comprises the ablation procedure control unit for controlling the ablation procedure, wherein the ablation procedure control unit is adapted to determine an actual position of the temperature probe during the ablation procedure and to guide the positioning of the temperature probe based on the actual position and the planned position of the temperature probe.

The ablation procedure control unit can be, for instance, a display for displaying the final ablation plan to a user during the ablation procedure to control the ablation procedure. The ablation procedure control unit can further comprise a medical procedure execution unit, like a medical robot, that is controlled by the ablation procedure control unit to execute the final ablation plan alone or in cooperation with a user.

Moreover, the ablation procedure control unit is adapted to determine an actual position of the temperature probe during the ablation procedure. For instance, the temperature probe can be adapted to comprise an actual position determination system, like an electromagnetic or optic actual position determination system, and the ablation procedure control unit can be adapted to determine the actual position of the temperature probe based on the signals provided by the actual position determination system. Alternatively, a grid template used during the ablation procedure can comprise a system that indicates when a probe is inserted through one of the grid points, wherein the control unit can be adapted to determine an actual position of the temperature probe based on the grid point indicated by the grid template. Preferably, the ablation procedure is executed under the guidance of an imaging system like an ultrasound system imaging the positioning of the temperature probe, wherein the control unit can be adapted to receive the images of the imaging system and to determine the actual position of the temperature probe based on the images.

The ablation procedure control unit is further adapted to guide the positioning of the temperature probe based on the actual position and the planned position. The guidance can, for instance, comprise providing an image of the region in which the temperature probe should be positioned on a display to a user and to indicate the actual and the planned position of the temperature probe on the image. The user can then directly navigate in accordance with the shown positions and ensure that the temperature probe is positioned correctly at the planned position. Moreover, the guidance can comprise controlling a robot based on the actual and the planed position so that the robot is operated such that the temperature probe is positioned at the planned position.

In a further aspect of the invention, a method for determining a planned position of a temperature probe during a planning for an ablation procedure is presented, wherein the method comprises the steps of a) providing an ablation plan, wherein the ablation plan comprises geometric information of an ablation region and of a protection region, wherein the protection region should be protected from ablation during the ablation procedure, wherein the geometric information comprises information on a position and shape of a respective region, b) determining a planned position of a temperature probe that should be positioned to protect the protection region during the ablation procedure, wherein the position of the temperature probe is determined based on the geometric information of the ablation region and of the protection region.

In a further aspect, a computer program for determining a position of a temperature probe for an ablation procedure is presented, wherein the computer program comprises program code means for causing the apparatus of claim 1 to carry out the steps of the method as defined in claim 14 when the computer program is executed on the apparatus.

It shall be understood that the apparatus of claim 1, the system of claim 12, the method of claim 14 and the computer program of claim 15 have similar and/or identical preferred embodiments, in particular as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 shows schematically and exemplarily an embodiment of an apparatus for determining a position of a temperature probe for an ablation procedure,
Fig. 2 shows schematically and exemplarily a function of such an apparatus, and
Fig. 3 shows a flowchart exemplarily illustrating an embodiment of a method for assisting and providing template treatment parameters for ablation treatment.

### DETAILED DESCRIPTION OF EMBODIMENTS

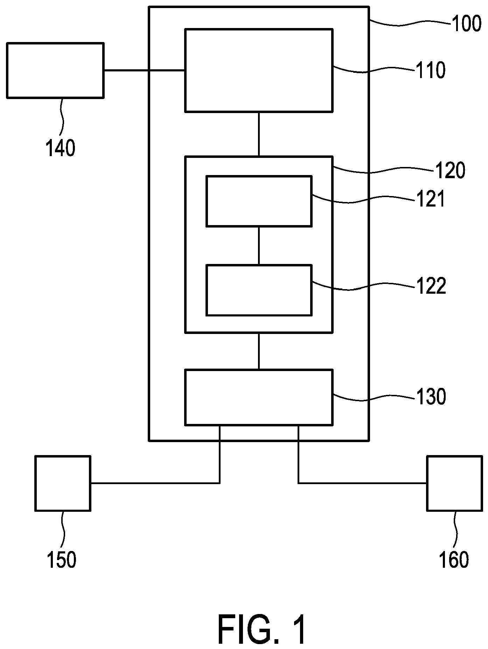

In Fig. 1 an apparatus for determining a planned position of a temperature probe for an ablation procedure is schematically and exemplarily shown in the context of an ablation procedure planning and guidance system comprising the apparatus for determining the planned position of the temperature probe. The ablation procedure planning system 100 comprises an ablation plan determination unit 110, the apparatus for determining the planned position of the temperature probe 120 and a final ablation plan providing unit 130.

The ablation plan determination unit 110 is adapted for determining an ablation plan for an ablation procedure based on input provided by an input providing unit 140. The input can refer, for instance, to a medical image of a region of interest of a patient, like a CT image, an ultrasound image, a PET image, etc., patient data like an age, a size, a weight, etc., information on the ablation system that should be used, like constructional characteristics of the ablation system, the functions provided by an ablation probe that should be used, etc.

The ablation plan determination unit 110 is then adapted in this embodiment to provide the user of the ablation procedure planning system 100 with the provided medical images of the region of interest, such that a user can indicate an ablation region referring to a region that should be ablated in the image and one or more protection regions that should be protected during the ablation procedure. In another embodiment, the ablation plan determination unit 110 can be adapted to automatically segment anatomical structures in the medical image and to identify automatically ablation regions and protection regions from the segmented structures. Based on the indicated ablation region and protection region the ablation plan determination unit 110 is adapted to determine an ablation plan for the ablation procedure.

The ablation plan comprises geometric information of the ablation region and of the protection region, preferably, comprises information on the position and shape of the ablation region and the protection region. The ablation plan can further comprise, for instance, a position of the ablation probe. Moreover, in this embodiment the ablation plan also comprises information on the position and structure of a grid template that should be used for positioning the ablation probe and the temperature probes during the ablation procedure. After the determination of the ablation plan, the ablation plan determination unit 110 provides the ablation plan to the apparatus 120. In particular, the ablation plan determination unit 110 provides the ablation plan to the ablation plan providing unit 121. The ablation plan providing unit 121 is in this embodiment a storage unit in which the ablation plan determination unit 110 stores the determined ablation plan. The ablation plan providing unit 121 is then adapted to provide the ablation plan or at least the geometric information of the respective regions to the temperature probe position determination unit 122.

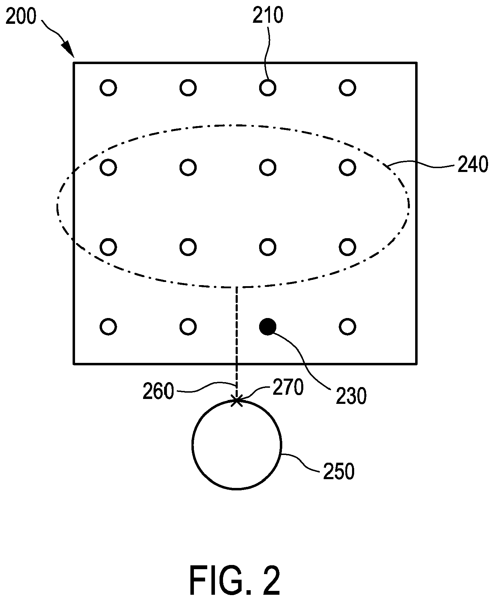

The temperature probe position determination unit 122 is adapted to determine a planned position of the temperature probe based on the geometric information of the ablation region and of the protection region determined by the ablation plan determining unit 110 and provided by the ablation plan providing unit 121. Moreover, in this embodiment the temperature probe position determination unit 122 is adapted to determine the planned position of the temperature probe further on the position of the grid template. An exemplary determination of the planned position of the temperature probe based on the geometric information of the ablation region and of the protection region and also on the position of the grid template should be further explained with reference to Fig. 2.

Fig. 2 shows schematically and exemplarily a grid template 200, an ablation region 240 and a protection region 250. The grid template 200, the ablation region 240 and the protection region 250 are schematically shown in Fig. 2 with respect to each other from a viewing direction perpendicular to the grid template 200. The grid template 200 comprises a plurality of grid points 210 through which an ablation probe and a temperature probe could be inserted into the patient. In the case shown here all grid points 210 are uniformly and evenly spaced on the grid template 200 in a periodic square-shaped distribution quadratic distribution. Moreover, the grid points 210 are constructed such that they allow to insert an ablation probe or a temperature probe in a perpendicular direction to the grid template 200. Based on the geometric information comprising the position and shape of the ablation region 240 and the protection region 250, the temperature probe position determination unit 122 is adapted to determine a shortest path 260 between the ablation region 240 and the protection region 250. The shortest path 260 describes in this embodiment a straight line between the ablation region 240 and the protection region 250 comprising a shortest distance between a boundary of the ablation region 240 and a boundary of the protection region 250. Further, the temperature probe position determination unit 122 is adapted to determine an intersection point 270 at which the shortest path 260 intersects the boundary of the ablation region 240. Moreover, the temperature probe position determination unit 122 is adapted to determine all possible paths of the temperature probe through the grid points 210 of the grid template 200. In this case these paths refer to a straight line through each grid point 210 perpendicular to the grid template 200. Further, the temperature probe position determination unit 122 is adapted to determine the possible path of the temperature probe that proceeds nearest to the intersection point 270. Moreover, the temperature probe position determination unit 122 determines a depth position of the temperature probe as closest point to the intersection point 270 along the previously determined nearest possible path of the temperature probe. Further for determining the planned position of the temperature probe the temperature probe position determination unit 122 then determines the grid point 230 belonging to the nearest possible path of the temperature probe. The planned position of the temperature probe then comprises the determined grid point 230 and the depth position. The temperature probe position determination unit 122 can then provide the determined planned temperature probe position to the final ablation plan providing unit 130.

The final ablation plan providing unit 130 can then combine the planned temperature probe position with the ablation plan to form the final ablation plan. Moreover, the final ablation plan providing unit 130 can comprise a verification unit for verifying and checking the final ablation plan. For instance, the verification unit can check the final ablation plan for consistency with predetermined goals of the ablation procedure or can check for inconsistencies in the final ablation plan. Then, the final ablation plan providing unit 130 can provide the final ablation plan to a display 150 for displaying the final ablation plan to a user. The user might then, for instance, accept, modify or reject the final ablation plan. If the user rejects or modifies the final ablation plan, the ablation procedure planning and guidance system 100 might again determine a final ablation plan in accordance with the above embodiment based on the modifications of the user, based on different input, or based on a differing ablation plan determination algorithm and again provide the respective resulting final ablation plan to the user on the display 150.

When the final ablation plan is finally accepted by the user, the final ablation plan providing unit 130 can provide the final ablation plan to an ablation procedure control unit 160 adapted for controlling the respective ablation procedure. In the here presented embodiment the ablation procedure control unit 160 is not a part of the system 100, but in other embodiments the ablation procedure control unit 160 can be a part of the system 100. For guiding the ablation procedure, the ablation procedure control unit 160 can, for instance, provide the final ablation plan to a user step by step, such that the user can follow the final ablation plan during the ablation procedure. Moreover, the ablation procedure control unit 160 might control one or more components of an ablation system in accordance with the final ablation plan. For instance, the ablation procedure control unit 160 might control the ablation probe in accordance with the final ablation plan. Further, the ablation procedure control unit 160 might be connected to a medical robot and control the medical robot, for instance, during the insertion of the temperature probe, in accordance with the final ablation plan.

Further, the ablation procedure control unit 160 can be specifically adapted to guide the positioning of the temperature probe. For instance, the ablation procedure control unit 160 can be adapted to determine an actual position of the temperature probe based on an actual position determination system provided in the temperature probe, or based on images like ultrasound images showing the temperature probe in the region in which it should be positioned. For guiding the user during the positioning of the temperature probe the ablation procedure control unit 160 can then show the actual position and the planned position on the display 150, for instance, indicated by a symbol in the image of the region in which the temperature probe should be positioned. The user can then position the temperature probe very accurately based on the indicated positions. Alternatively, if the temperature probe is positioned by a medical robot, the ablation procedure control unit 160 can be adapted to control the medical robot based on the actual and the planned position of temperature probe to position the temperature probe at the planned position.

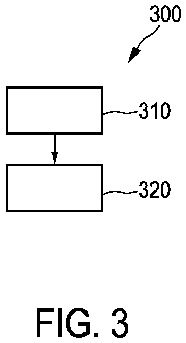

Fig. 3 shows schematically and exemplarily a flowchart of a method for determining a planned position of a temperature probe during a planning of an ablation procedure. The method 300 comprises a first step 310 of providing an ablation plan. The ablation plan comprises geometric information of an ablation region and of a protected region, wherein the geometric information comprises information on the position and shape of the respective regions. In a second step 320, a planned position of a temperature probe is determined based on the geometric information of the ablation region and of the protection region. The position of the temperature probe can be, for instance, determined as explained above with respect to Fig. 2.

The invention as described above relates preferably to the field of thermal ablation in interventional oncology and can address a need for planning and guiding during a placement of temperature probes to aid a user in monitoring the safety and effectiveness of an ablation procedure. Generally, percutaneous thermal ablation is an interventional cancer treatment option that has seen significant increase in adoption in the past decade and is predicted to continue to grow at a compound annual growth rate of 8 % to 10 % through 2024. Thermal ablation can be delivered using various ablation modalities, including radiofrequency, microwaves, high intensity focused ultrasound, focal laser ablation, irreversible electroporation, cryoablation, etc.

In clinical practice, these ablation procedures consist of placing one or more ablation applicators, i.e. ablation probes, inside or near a target region, i.e. an ablation region, for instance, with the help of image guidance. Typically, a physician places needle-like probes while inspecting real-time ultrasound or interventional radiology images. The probes are mainly based on information provided by the manufacturer, results and clinical trials, and/or personal experience. A use of more advanced ablation therapy planning systems to plan an ablation and guide needle placement is not widely spread due to their limited availability. Presently, it is common practice to place one or more temperature probes to monitor the safety and effectiveness of the ablation, wherein the placement of these probes is again based on the physician's knowledge and experience without using more dedicated support.

Since the placement of the temperature probes is currently completely based on knowledge, skill and experience of the treating physician, placements vary between physicians and may be suboptimal in some procedures. Consequently, organs at risk, i.e. protection regions, that are supposed to be protected, can still be damaged from excessively low/high temperatures despite the use of temperature probes.

This invention proposes, for instance, an ablation procedure planning and guidance system that includes an automatic planning method that allows to determine a planned position of temperature probes that can prevent exceedingly high/low temperatures in organs at risk, i.e. protection regions. Based on the determined planned position an ablation procedure planning and guidance system can then provide guidance to a user to support an accurate placement of the temperature probes.

While the planning and guiding for a temperature probe placement as proposed in this invention, i.e. the apparatus for determining a position of a temperature probe, can be used in a dedicated ablation quality assurance system, the invention can also be used in addition to or integrated with a more advanced or sophisticated ablation procedure planning and guidance system. Such an ablation procedure planning and guidance system can allow a user to delineate a target region, i.e. ablation region, and organs at risk, i.e. protection regions, in medical images to be considered during the planning process. This planning process can then be supported by allowing the user to define intended locations and ablation parameters for one or more ablation probes and can visualize the associated ablation zones, temperature fields or thermal doses received by the patient. In addition, in some systems the user can assess the quality of the plans by inspecting various quantitative parameters such as, for instance, the percentage of tissue ablated in the ablation region and/or a protection region.

An embodiment of the here described invention includes automatically determining a planned position of a temperature probe in order to protect an organ at risk, i.e. a protection region, from accidental ablation. Preferably, this automated determination of the planned position can be computed based on geometrical information available to the system and thus remains independent of the ablation modality in use. In an example, a computer algorithm might be used that receives as an input a segmentation, i.e. position and shape, of the ablation region, a segmentation, i.e. position and shape, of the protection region and a position of a grid template. The algorithm might then first determine all potential needle paths originating from the grid template 200, and the shortest path between the segmented ablation region and the segmented protection region. Further, the algorithm might determine an intersection point of the shortest path and the protection region. Then, the planned position of the temperature probe might be determined by determining the potential needle path that proceeds closest to the intersection point and a position closest to the intersection point on the closest potential needle path as position of the temperature probe. The algorithm can then return the position of the temperature probe as an output.

Such an algorithm might guarantee that the temperature probe is positioned closer to the ablation region than to the protection region when the grid template 200 is positioned such that no potential needle tracks, i.e. possible paths of the temperature probe, can be found through the protection region. In such a case the ablation procedure will cause more pronounced temperature changes at the proposed temperature probe position than in the protection region. As such, this strategic positioning can be used to prevent extreme temperatures in the protection region. The potential needle paths, i.e. possible paths of the temperature probe, in the temperature probe placement algorithm cover all possible needle positions, i.e. temperature probe positions, without puncturing a protection region that should not be punctured. Whether a protection region should be avoided or not may be configured in the ablation plan.

The temperature probe position determination algorithm may be invoked between a single/selected ablation region and a selected protection region, between all ablation regions and all protection regions provided in an ablation plan and/or between specific ablation regions and protection regions provided in the ablation plan. Moreover, the temperature probe position determination algorithm may further be invoked between one or more ablation regions and one or more protection regions upon explicit request by a user during the planning or during the interventional procedure itself, or might be automatically be invoked based on the ablation plan.

For effective protection of the protection region it is advantageous if during the ablation procedure it is possible to process the temperature measurements from the temperature probes in real-time. The real-time processing can include, for instance, a comparison against a threshold temperature that may be determined for an individual temperature probe or that may be identical for all temperature probes in use. In addition, the temperature threshold might be used for a temperature alarm, wherein the threshold temperatures may be fixed, configurable as part of the ablation plan and/or modifiable upon explicit request by the user during the ablation procedure.

An ablation procedure planning and guidance system into which the above described temperature probe position determination algorithm is integrated can provide, for instance, image guidance to achieve placement that is in line with the determined and proposed positions. This guidance can rely, for instance, on the same guidance mechanism that supports the user in placing ablation probes. Such guidance can help to guarantee an accurate placement of the temperature probes, which might be crucial for a quality assurance.

Further, the ablation procedure planning and guidance system can be capable of pre-computing/simulating an estimated temperature development of the tissue throughout the ablation procedure. This offers the possibility for monitoring the ablation procedure using the temperature probes. For example, an ablation procedure control system might be adapted to compare measured and pre-computed temperatures displayed in tables, charts, etc. in real-time. Moreover, such an ablation procedure control system may process the difference between measured and pre-computed temperatures in real-time and sound an alarm if the difference exceeds a predefined threshold. This threshold can be fixed, configurable or modifiable similarly to the temperature threshold as described above. In addition, the estimated temperature development may be used as additional input for the temperature probe position determination algorithm, wherein, for instance, the algorithm may select a position along a potential needle path, i.e. possible path of a temperature probe, near the protection region at which an estimated maximum temperature occurs. Such an approach might be advantageous when dealing with potential heat sinks near a protection region, but might also be more specific for an ablation modality in use.

Although in above embodiments the apparatus was provided as being part of an ablation planning and guidance system, in other embodiments the apparatus can be a stand-alone system or a system integrated in an ablation procedure control unit.

Although in above embodiments the planned position of the temperature probe was determined based on the geometric information of the ablation region and the protection region and the position of the grid template, in other embodiments the temperature probe position determination unit can be adapted to determine the planned position of the temperature probe only based on the geometric information. Moreover, in other embodiments the ablation plan providing unit can be adapted to provide an ablation plan comprising further an expected temperature development during the ablation procedure, wherein in this case the temperature probe position determination unit can be adapted to determine the planned position of the temperature probe based on the geometric information of the ablation region, the geometric information of the protection region, and the expected temperature development. Also the grid template can be additionally taken into account.

Although in above embodiments the temperature probe position determination unit only determines a planned position for the temperature probe, in other embodiments the temperature probe position determination unit can be adapted to further determine a temperature threshold, wherein the temperature threshold can be determined such that if the temperature probe measures only temperatures below or above the temperature threshold during the ablation procedure, it can be assumed that the protection region is not affected by the ablation procedure, whereas if the temperature probe measures temperatures above or below the temperature threshold, an alarm might be sounded to inform the user that the protection region might be affected by the ablation procedure.

Although in the above embodiment the ablation probe provided a heating of the ablation region, in other embodiments the ablation probe can be a cryoablation probe and provide a cooling of the ablation region.

Other variations to the disclosed embodiments can be understood and effected by those skilled in the art in practicing the planned invention, from the study of the drawings, the disclosure, and the appendant claims.

In the claims, the word "comprising" does not exclude other elements or steps, and the indefinite article "a" or "an" does not exclude a plurality.

A single unit or device may fulfill the functions of several items recited in the claims. The mere fact that certain measures are recited in mutually different dependent claims does not indicate that a combination of these measures cannot be used to advantage.

Procedures like the providing of the ablation plan or the determination of the temperature probe positions performed by one or several units or devices can be performed by any other number of units or devices. The procedures and/or the operations of the apparatus can be implemented as program code means of a computer program and/or as dedicated hardware.

A computer program may be stored/distributed on a suitable medium, such as any optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The invention refers to an apparatus allowing to determine a planned position of a temperature probe during a planning for an ablation procedure. The apparatus comprises an ablation plan providing unit for providing an ablation plan, wherein the ablation plan comprises geometric information of an ablation region and of a protection region. The protection region should be protected from ablation. The geometric information comprises information on a position and shape of a respective region. A temperature probe position determination unit is adapted to determine a planned position of the temperature probe, wherein the planned position of the temperature probe is determined based on the geometric information of the ablation region and of the protection region. The apparatus allows to increase the protection of regions that should be protected, like a specific organ, such that the security of an ablation procedure for a patient can also be increased.

## Claims

1. An apparatus for determining a position of a temperature probe during a planning for an ablation procedure, wherein the apparatus comprises:
- an ablation plan providing unit (121) for providing an ablation plan, wherein the ablation plan comprises geometric information of an ablation region (240) and of a protection region, wherein the protection region (250) should be protected from ablation during the ablation procedure, wherein the geometric information comprises information on a position and shape of a respective region,
- a temperature probe position determination unit (122) for determining a planned position of the temperature probe that should be positioned to protect the protection region (250) during the ablation procedure, wherein the planned position of the temperature probe is determined based on the geometric information of the ablation region (240) and of the protection region.

2. The apparatus according to claim 1, wherein the temperature probe position determination unit (122) is adapted to determine a shortest path (260) between the ablation region (240) and the protection region (250) and to determine the planned position of the temperature probe along the shortest path (260).

3. The apparatus according to claims 1 or 2, wherein the ablation plan further comprises a position of a grid template (200) comprising a plurality of grid points through which a temperature probe can be inserted, wherein the temperature probe position determination unit (122) is adapted to further determine the planned position of the temperature probe based on the position of the grid template.

4. The apparatus according to claim 3, wherein the temperature probe position determination unit (122) is adapted to determine a planned position of the temperature probe comprising a grid point of the grid template (200) through which the temperature probe should be positioned.

5. The apparatus according to claim 4, wherein the temperature probe position determination unit (122) is adapted to determine an intersection point (270) between the shortest path (260) and the protection region, and to determine as planned position of the temperature probe a position of a nearest grid point with respect to the intersection point.

6. The apparatus according to any of the previous claims, wherein the ablation plan further comprises an estimated temperature development during the course of the ablation procedure for an affected region comprising the target region and the protection region, wherein the temperature probe position determination unit (122) is adapted to determine the planned position of the temperature probe further based on the estimated temperature development.

7. The apparatus according to claim 6, wherein the temperature probe position determination unit (122) is adapted to determine the planned position of the temperature probe based on the expected temperature development such that expected temperature changes during the ablation procedure at the planned position of the temperature probe are higher than in the protected region.

8. The apparatus according to claim 6, wherein the temperature probe position determination unit (122) is adapted to determine the planned position of the temperature probe as a position for which the highest temperatures are expected within a safety margin around the protected region.

9. The apparatus according to any of the proceeding claims, wherein the temperature probe position determination unit (122) is further adapted to provide a threshold temperature based on a determined planned position of the temperature probe, wherein the threshold temperature is determined such that, in a case in which the tissue is ablated using heat, if temperatures below the threshold temperature are measured by the temperature probe positioned at the determined position, tissue in the protection region (250) will not be damaged, or such that, in a case in which the tissue is ablated using cold, if temperatures above the threshold temperature are measured by the temperature probe positioned at the determined position, tissue in the protection region (250) will not be damaged.

10. The apparatus according to claim 9, wherein the ablation plan further comprises an estimated temperature development during the course of the ablation procedure for an affected region comprising the target region and the protected region, wherein the temperature probe position determination unit (122) is adapted to determine the temperature threshold further based on the estimated temperature development.

11. The apparatus according to any of the previous claims, wherein the ablation plan further comprises information on a sensitivity of the protection region, wherein the temperature probe position determination unit (122) is further adapted to determine the planned position of the temperature probe based on the sensitivity information.

12. An ablation procedure planning and guidance system for planning and guiding an ablation procedure, wherein the system comprises:
- an ablation plan determination unit (110) for determining an ablation plan comprising geometric information on an ablation region (240) and a protection region,
- an apparatus according to claim 1 for determining a planned position of a temperature probe for the ablation procedure, and
- a final ablation plan providing unit (130) for providing a final ablation plan comprising the ablation plan and the positions of the temperature probe to an ablation procedure control unit (160) for controlling the ablation procedure.

13. An ablation procedure planning and guidance system according to claim 12, wherein the system further comprises an ablation procedure control unit (160) for controlling the ablation procedure, wherein the ablation procedure control unit is adapted to determine an actual position of the temperature probe during the ablation procedure and to guide the positioning of the temperature probe based on the actual position and the planned position of the temperature probe.

14. A method for determining a position of a temperature probe during a planning for an ablation procedure, wherein the method comprises the steps of:
- providing an ablation plan, wherein the ablation plan comprises geometric information of an ablation region (240) and of a protection region, wherein the protection region (250) should be protected from ablation during the ablation procedure, wherein the geometric information comprises information on a position and shape of the respective region,
- determining a position of a temperature probe that should be positioned to protect the protection region (250) during the ablation procedure, wherein the position of the temperature probe is determined based on the geometric information of the ablation region (240) and of the protection region.

15. A computer program for determining a position of a temperature probe for an ablation procedure, wherein the computer program comprises program code means for causing the apparatus of claim 1 to carry out the steps of the method as defined in claim 13 when the computer program is executed on the apparatus.
